# EUROPEAN PATENT SPECIFICATION

(11) **EP 3 148 596 B1**
(45) Date of publication and mention of the grant of the patent: **26.09.2018**
(21) Application number: 15726085.2
(22) Date of filing: 25.05.2015
(51) Int. Cl.: A61L 9/12

(54) **DIFFUSER OF SCENTS WITH FACILITATED ACTIVATION**
DIFFUSOR VON DUFTSTOFFEN MIT VEREINFACHTER AKTIVIERUNG
DIFFUSEUR DE PARFUMS POURVU D'UNE ACTIVATION FACILITÉE

(30) Priority: 28.05.2014 IT MO20140154
(43) Date of publication of application: 05.04.2017
(73) Proprietor: FD Packaging Società a Responsibilità Limitata, 41019 Soliera (MO) (IT)
(72) Inventor: FREDDI, Antonio, I-42046 Reggiolo (IT)
(74) Representative: Corradini, Cesare
(86) International application number: PCT/EP2015/061489
(87) International publication number: WO 2015/181103

(56) References cited:
- EP-A1- 1 797 905
- WO-A1-2013/076033
- DE-U1-202004 021 681
- US-A1- 2012 108 888

## Description

The present invention relates to a diffuser of scents with facilitated activation.

Diffusers of scents are known for environments, which consist of packages that are substantially constituted by a heat-formed tray for collecting the scented substance which is closed by a breathable membrane. Since the diffusion of the scent is activated when the breathable membrane is in contact with the scented substance, the tray generally has two separate volumes, one of which is for collection, in which the scented substance is kept segregated until the moment of use, and one is for expansion at the breathable membrane, into which the scented substance is poured at the moment of use. The fluid connection between such volumes is blocked by temporary barrier systems which are adapted to be deactivated by the user in order to allow the diffusion of the scent.

Different forms exist of packages which are structured as described above.

In particular, from WO2013/076033 by the same Applicant, a package is known in which there is a thermoformed main body which defines a collection tray for the scented substance and an expansion chamber for the diffusion of the scent facing the breathable membrane, which are connected by way of a passage channel which is temporarily closed.

Furthermore, there is a separation film interposed between the main body and the breathable membrane, which adheres to the main body at a first closed perimetric band around the containment chamber, a second closed perimetric band around the expansion seat, and a connection region between the tray and the chamber. The separation film has one or more slots at the expansion chamber in order to allow the scented substance to come into contact with the breathable membrane in use.

At the connection region there is a weaker connection than the other areas of adhesion, which is obtained by way of suitable techniques of heat-sealing or gluing, and which can be forced so as to detach the film from the main body so as to open the passage channel for the outflow of the scented substance to the expansion chamber.

In a preferred embodiment the expansion chamber is arranged on the peripheral region of the collection tray, and the connection region is defined by a portion of the first perimetric band.

In use, by pressing the package at the collection tray, the pressure exerted by the scented substance internally on the first band of adhesion causes the yielding of the connection region at the passage channel and, thus, the separation of the film from the wall of the main body, allowing the scented substance to flow into the expansion seat for the diffusion of the scent.

This conventional package is not devoid of drawbacks, among which is the fact that it is not ensured that the user will obtain a correct and complete opening of the chambers solely by way of pressure exerted on the tray, which needs to be pressed with a certain firmness and thus is inconvenient to use for some users.

Also, controlling the process of adhering the film to the main body so as to obtain a region that is weaker at the passage channel requires experience and sophisticated technologies.

Furthermore, such method of activation causes an instantaneous opening and a sudden flow of the scented substance toward the expansion seat.

Finally, since the passage opening of the scented substance tends to reduce in size in that, after the initial eructation owing to the pressure exerted, the film tends to return toward the wall of the main body, with consequent risk that a residue of scented substance remains in the collection tray, resulting in a waste of product and a reduction of the duration of the scenting effect of the diffuser. Such drawbacks are even more accentuated as the viscosity of the scenting substance increases.

The aim of the present invention is to eliminate the above mentioned drawbacks in the background art by providing a diffuser of scents with facilitated activation which makes it possible to ensure a correct and complete activation, without requiring particular effort or ability of the users.

Within this aim, an object of the present invention is to not require sophisticated manufacturing techniques or particular checks during manufacture.

Another object of the present invention is to allow a gradual activation and, thus, a regular flow of the scented substance.

Another object of the present invention is to make it possible to transfer all the scented substance to the expansion seat, thus preventing unwanted waste and obtaining the maximum efficacy and duration of the diffuser.

Another object of the present invention is to provide a simple structure that is easy and practical to implement, safe in use and effective in operation, and low cost.

This aim and these objects are achieved by the present diffuser of scents with facilitated activation, which comprises a main body provided with a supporting wall in which there are, in a recessed arrangement, a tray for collecting a scented substance and an expansion chamber, a separation film which is superimposed on said main body so as to close said collection tray and said expansion chamber and is provided with at least one through slot at said chamber, and a breathable membrane superimposed on said separation film at least at said at least one slot, the separation film being associated with and adhering to the supporting wall at least at a first perimetric band which is external to the collection tray, at a second perimetric band which is external to the expansion chamber and at at least one region for connection between said tray and said chamber, characterized in that it comprises retractor means which are accommodated at one or both of the collection tray and the expansion chamber in order to detach said separation film and said supporting wall at said at least one connection region so as to define at least one channel for the passage of the scented substance, which are activatable by way of a pressure applied from the outside at said one or both of the collection tray and the expansion chamber.

Further characteristics and advantages of the present invention will become better apparent from the detailed description of a preferred, but not exclusive, embodiment of a diffuser of scents with facilitated activation, which is illustrated by way of non-limiting example in the accompanying drawings wherein:
Figure 1 is an exploded perspective view of a diffuser of scents with facilitated activation, according to the invention;
Figure 2 is a perspective view from below of the diffuser of scents in Figure 1 in the assembled configuration;
Figure 3 is a cross-sectional perspective view of the diffuser of scents in Figure 2;
Figures 4 and 5 are cross-sectional views of the diffuser of scents according to the invention, with the retractor means in the inactive configuration and in the operating configuration, respectively;
Figures 6 and 7 are enlarged-scale detail views of the region of the passage channel, respectively, in Figures 4 and 5;
Figures 8 and 9 are perspective views of alternative embodiments of the retractor means.

With reference to the figures, the reference numeral 1 generally designates a diffuser of scents with facilitated opening which can be used, for example, in enclosed environments, of the type of rooms, premises or vehicle cabins.

The diffuser 1 comprises a main body 2 comprising a supporting wall 3 in which there are, in a recessed arrangement, a tray 4 for collecting a scented substance S and an expansion chamber 5 for the diffusion of the scent deriving from this substance.

The main body 2 can be obtained, for example, by way of conventional processes of molding or plastic deformation of a deformable material, for example plastics or aluminum, in order to then be deformable in use at the collection tray 4 and/or at the expansion chamber 5.

Preferably the volume of the expansion chamber 5 and of the collection tray 4 are mutually correlated in a ratio comprised between 20% and 150%.

The scented substance S can be of the type of conventional liquid or fluid scents and is introduced a in quantity that is generally comprised between 1 and 20 milliliters.

The diffuser 1 also comprises a separation film 6 which is superimposed on the main body 2 so as to close the collection tray 4 and the expansion chamber 5 and is provided with one or more through slots 7, which are defined at the chamber.

The separation film 6 is associated with and adherent to the wall 3 for supporting the main body 2 at least at a first perimetric band 8 which is external to the collection tray 4, at a second perimetric band 18 which is external to the expansion chamber 5 and at at least one region 9 for connection between the tray and the chamber.

Such adhesion areas or "closing seams" can be obtained by way of conventional heat-sealing or gluing processes.

The separation film 6 is made of conventional materials which are not permeable to the scent.

In this manner the scented substance S remains confined in the volume of the collection tray 4 until the moment of use.

In the version shown, the diffuser 1 has the expansion chamber 5 defined along the perimeter of the collection tray 4 and the first band 8 extends between the tray and the chamber. In this case the connection region 9 is defined so as to be integral with a portion of the first band 8.

Alternative embodiments are not ruled out in which, for example, the collection tray 4 and the expansion chamber 5 are arranged side-by-side and the connection region 9 is interposed between them.

The diffuser 1 comprises, further, a breathable membrane 10 which faces the outside environment and is associated by superimposition with the separation film at least at a portion of the expansion chamber 5, i.e. at least at the slots 7. Preferably the breathable membrane 10 has a shape in plan view which matches that of the main body 2, so as to cover it uniformly.

The breathable membrane 10 is made of a microporous material with a variable passage porosity between 0.01 and 5 micrometers, preferably selected from between 0.015 and 0.5 micrometers, of natural or synthetic origin. For example materials based on polyolefins or polyethylene such as Teslin®, Solupor® or the like are suitable. The breathable membrane 10 allows the passage of larger scent molecules than the scent molecules dispensed by conventional breathable or osmotic membranes.

In use, the scented substance S poured into the expansion chamber 5 comes into contact with the breathable membrane 10 through the slots 7 and diffuses its scent into the outside environment.

According to the invention the diffuser 1 comprises, further, retractor means 11 accommodated at one or both of the collection tray 4 and the expansion chamber 5.

The retractor means 11 are adapted to separate the separation film 6 from the supporting wall 3 at the connection region 9 so as to define a channel 12 for the passage of the scented substance S from the collection tray 4 to the expansion chamber 5 upon use.

The retractor means 11 are activatable by way of a pressure applied from the outside at the region of the main body 2 in which they are accommodated.

Thanks to the presence of the retractor means 11 it is substantially not necessary to modify (but only slightly) the resistance of the adhesion between the separation film 6 and the supporting wall 3 at the connection region 9 with respect to the bands 8 and 18.

Furthermore the retractor means 11 facilitate the use of the diffuser 1, in that it is sufficient to apply a light compression in order to activate the dispensing of the scent, and the complete and correct opening is ensured of the passage channel 12 as is the keeping of the opening thereof so as to allow the passage of all of the scented substance S into the expansion chamber 5.

In the embodiment shown the retractor means 11 are accommodated inside the collection tray 4, but the possibility is not ruled out of their being positioned at the expansion chamber 5.

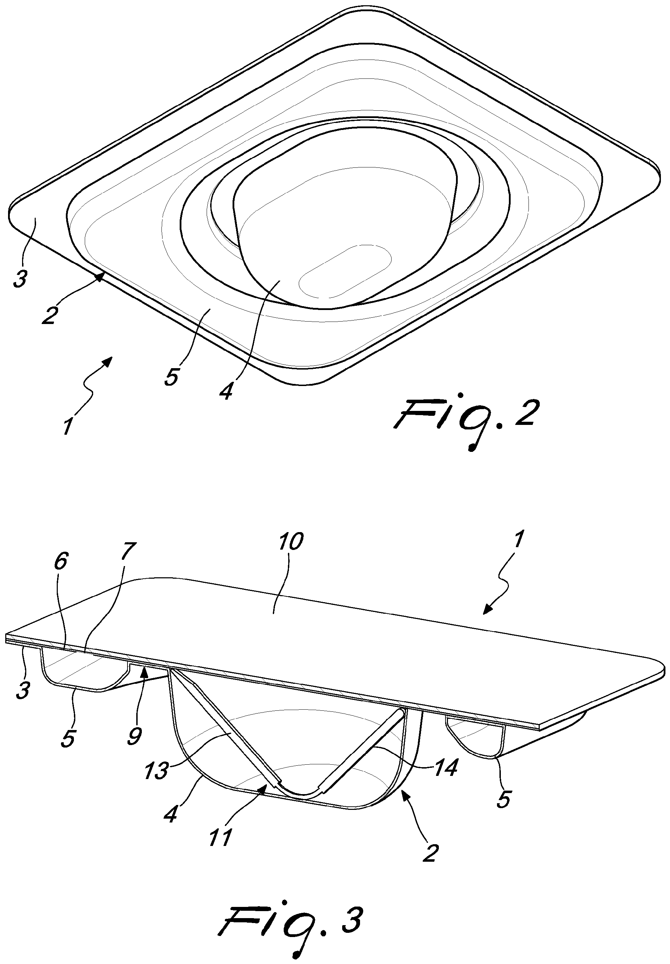

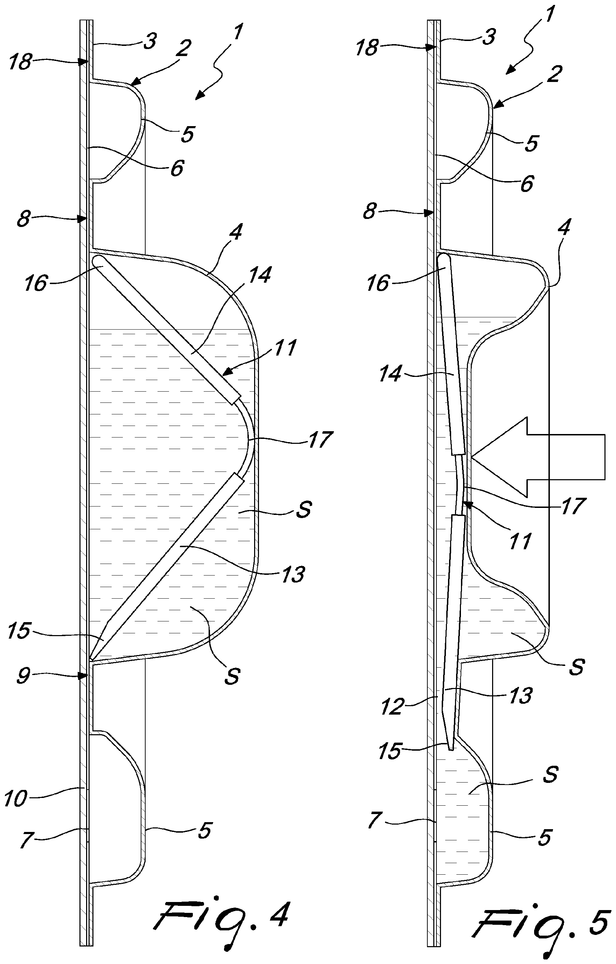

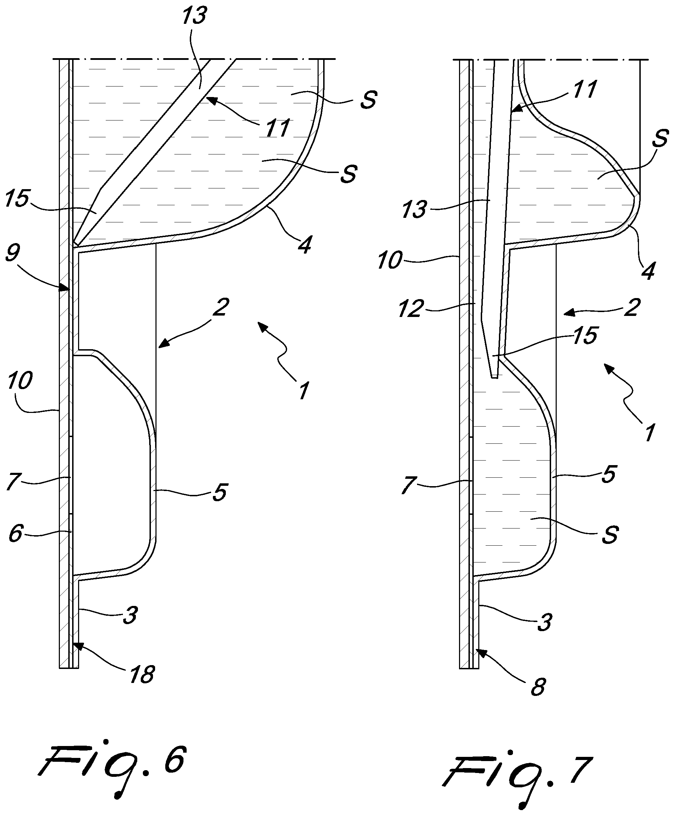

Preferably, the retractor means 11 comprise at least one separator 13 and at least one abutment element 14, which are mutually articulated. The elements 13 and 14 are adapted to pass from an inactive configuration (Figures 3, 4 and 6), in which they are completely accommodated in the collection tray 4, with the end of the separator 13 arranged at the connection region 9, and are mutually inclined so as to define a first acute angle, to an operating configuration (Figures 5 and 7), in which they are mutually retracted so as to define a second acute angle, greater than the first, and with the separator 13 inserted so that it passes through the connection region 9 between the separation film 6 and the supporting wall 3 so as to open the passage canal 12 and the abutment element 14 resting against the inner wall of the collection tray 4, following a pressure applied from the outside at their region of articulation.

The separator 13 is linear in shape, with one end coupled to the abutment element 14 and the opposite end free and tapered in shape so as to define a sort of wedge 15 which is adapted to slip in between the separation film 6 and the supporting wall 3.

The abutment element 14 is linear in shape, with one end coupled to the separator 13 and the opposite end free and rounded 16, so as to not damage the wall of the collection tray 4.

The retractor means 11 are provided in a single body and comprise a connection portion 17 made of deformable material interposed between the elements 13 and 14, which allows their articulation and is in contact with the bottom of the collection tray 4. The possibility is not ruled out however of the elements 13 and 14 being made as separate components and mutually articulated in order to allow their movement.

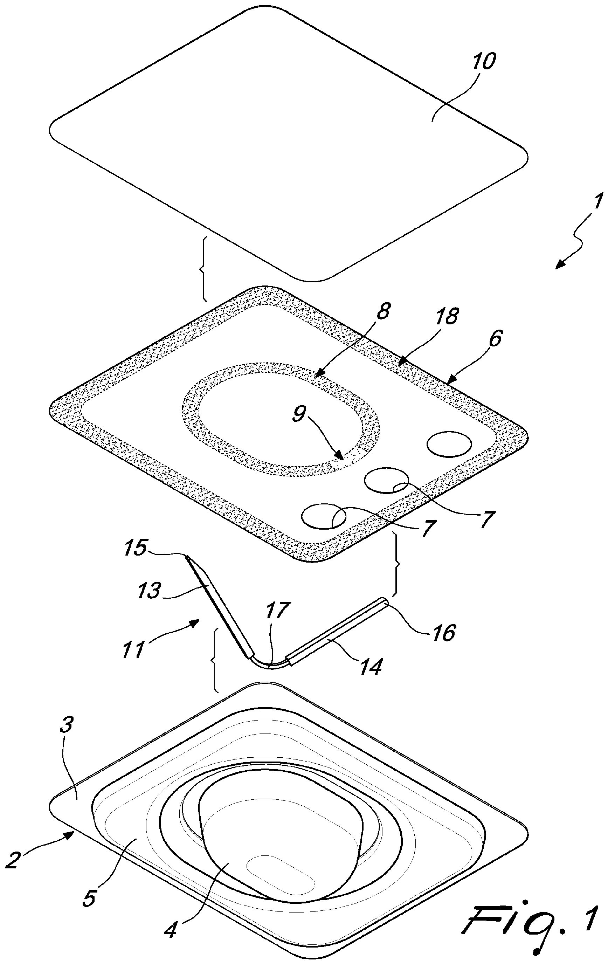

In a first embodiment (Figures 1-8) the retractor means 11 are constituted by a sort of compass, having a separator 13 and an abutment element 14.

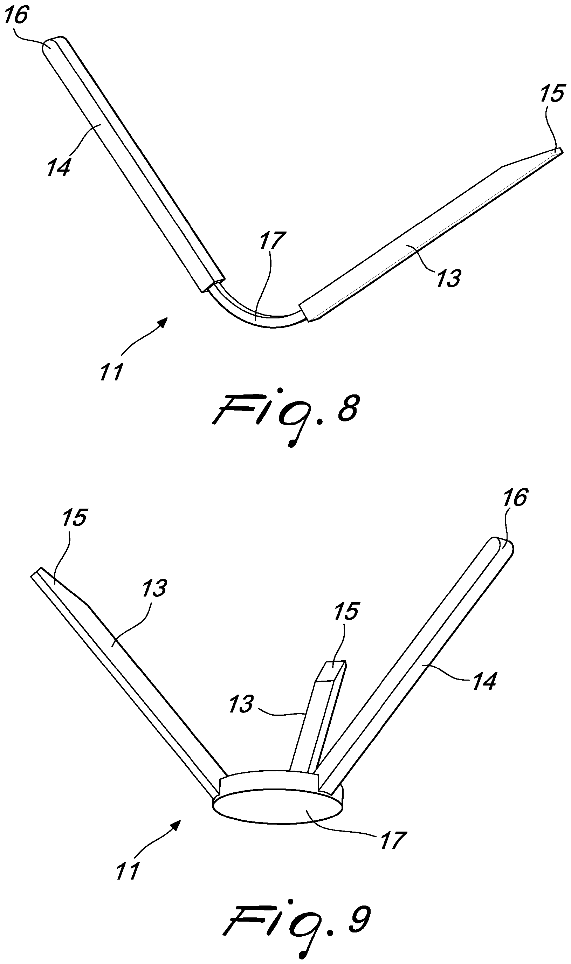

In a second embodiment (Figure 9) the retractor means 11 have an abutment element 14 and two separators 13 which are arranged symmetrically with respect to the arrangement of the abutment element. In this case, for each separator 13 there must be a respective connection region 9 so as to define a corresponding channel 12 for the passage of the scented substance S into the expansion chamber 5 for use.

However, alternative embodiments are not ruled out which have a different number of elements 13 and/or 14; in such case, more separators 13 are provided.

The method for manufacturing the diffuser 1 involves the following steps:
- a step of forming the main body 2 as described above,
- a step of introducing into the collection tray 4 a predefined volume of scented substance S,
- a first step of juxtaposing the separation film 6 on the main body 2 so as to close the collection tray 4 and the expansion chamber 5, with the slots 7 arranged at the expansion chamber 5,
- a first step of adhesion of the separation film 6 and of the supporting wall 3 at least at the first band 8, at the second band 18 and at the connection region 9,
- a second step of juxtaposing the breathable membrane 10 on the separation film, and
- a second step of adhesion of the breathable membrane 10 to the separation film.

The method according to the invention comprises, further, a step of positioning the retractor means 11 inside one or both of the collection tray 4 and the expansion chamber 5, which is carried out prior to the first step of juxtaposition.

Such step of positioning can be carried out, indifferently, before, during or after the step of introducing the scented substance S.

Operation of the present invention is the following.

When using the diffuser 1 the user applies a pressure to the main body 2 at the retractor means 11, directly or by way of inserting the diffuser into adapted supports of conventional type.

In this manner, the transition is actuated of the retractor means 11 from the inactive configuration to the operating configuration as described above, so as to allow the transfer of the scented substance S into the expansion chamber 5 and thus the dispensing of the scent through the breathable membrane 10.

By positioning the diffuser 1 with a vertical arrangement and with the passage channel 12 downwardly, such transfer occurs spontaneously.

In practice it has been found that the invention as described achieves the intended aim and objects and, in particular, attention is drawn to the fact that the presence of the retractor means makes it possible to facilitate the activation of the dispensing of the scent and ensures a correct and complete transfer of the scented substance, so as to avoid waste thereof and optimize the efficacy and duration of the diffuser.

Furthermore, the diffuser according to the invention simplifies the process of manufacture, because it does not require special checks on the step of adhering the separation film to the supporting body.

The invention, thus conceived, is susceptible of numerous modifications and variations, all of which are within the scope of the appended claims.

Moreover, all the details may be substituted by other, technically equivalent elements.

In practice the materials employed, as well as the contingent dimensions and shapes, may be any according to requirements without for this reason departing from the scope of protection claimed herein.

The disclosures in Italian Patent Application No. MO2014A000154 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A diffuser (1) of scents with facilitated activation, which comprises:
- a main body (2) provided with a supporting wall (3) in which there are, in a recessed arrangement, a tray (4) for collecting a scented substance (S) and an expansion chamber (5),
- a separation film (6) which is superimposed on said main body (2) so as to close said collection tray (4) and said expansion chamber (5) and is provided with at least one through slot (7) at said chamber, and
- a breathable membrane (10) superimposed on said separation film (6) at least at said at least one slot (7),
the separation film (6) being associated with and adhering to the supporting wall (3) at least at a first perimetric band (8) which is external to the collection tray (4), at a second perimetric band (18) which is external to the expansion chamber (5) and at at least one region (9) for connection between said tray and said chamber,
**characterized in that** it comprises retractor means (11) which are accommodated at one or both of the collection tray (4) and the expansion chamber (5) in order to detach said separation film (6) and said supporting wall (3) at said at least one connection region (9) so as to define at least one channel (12) for the passage of the scented substance (S), which are activatable by way of a pressure applied from the outside at said one or both of the collection tray (4) and the expansion chamber (5).

2. The diffuser (1) according to claim 1, **characterized in that** said retractor means (11) comprise at least one separator (13) and at least one abutment element (14), which are mutually articulated and are adapted to pass from an inactive configuration, in which they are accommodated completely in one or both of the collection tray (4) and the expansion chamber (5), to an operating configuration, in which they are mutually retracted, with the at least one separator (13) inserted so as to pass through the connection region (9) between the separation film (6) and the supporting wall (3) so as to open the passage channel (12), following the application of said pressure from the outside.

3. The diffuser (1) according to claim 2, **characterized in that** said at least one separator (13) has a tapered free end (15).

4. The diffuser (1) according to claim 2, **characterized in that** said at least one abutment element (14) has a rounded free end (16).

5. The diffuser (1) according to one or more of the preceding claims, **characterized in that** said retractor means (11) comprise at least two abutment elements (14).

6. The diffuser (1) according to one or more of the preceding claims, **characterized in that** said retractor means (11) comprise at least two separators (13), at least two respective connection regions (9) being provided.

7. The diffuser (1) according to one or more of the preceding claims, **characterized in that** said expansion chamber (5) is arranged on the peripheral region of said collection tray (4) and **in that** said first perimetric band (8) extends between said collection tray (4) and said expansion chamber (5), the connection region (9) being defined so as to be integral with a portion of the first perimetric band (8).

8. A method for manufacturing a scent diffuser (1), comprising:
- a step of forming a main body (2) comprising a supporting wall (3) in which there are, in a recessed arrangement, a tray (4) for collecting a scented substance (S) and an expansion chamber (5),
- a step of introducing into said collection tray (4) a predefined volume of scented substance (S),
- a first step of juxtaposing a separation film (6) on said main body (2) so as to close said collection tray (4) and said expansion chamber (5), the separation film (6) being provided with at least one through slot (7) located at the expansion chamber (5),
- a first step of adhesion of said separation film (6) and of said supporting wall (3) at least at a first perimetric band (8) which is external to said collection tray (4), at a second perimetric band (18) which is external to said expansion chamber (5), and at at least one connection region (9) between said tray and said chamber,
- a second step of juxtaposing a breathable membrane (10) on said separation film (6) at least at said slot (7),
- a second step of adhesion of said breathable membrane (10) and of said separation film (6),
**characterized in that** it comprises a step of placing retractor means (11) within one or both of the collection tray (4) and the expansion chamber (5), which is performed prior to said first juxtaposition step, the retractor means (11) being adapted to detach the separation film (6) and the supporting wall (3) at said at least one connection region (9) so as to define at least one channel (12) for the passage of the scented substance (S) and being activatable by way of a pressure applied from the outside.

## Patentansprüche

1. Diffuser (1) für Düfte mit erleichterter Aktivierung, der Folgendes umfasst:
- einen Hauptkörper (2), der mit einer tragenden Wand (3) versehen ist, in der in einer eingesenkten Anordnung eine Wanne (4) zum Aufnehmen einer mit Duft versehenen Substanz (S) und eine Ausdehnungskammer (5) vorhanden sind,
- eine Trennfolie (6), die über dem Hauptkörper (2) angeordnet ist, so dass die Aufnahmewanne (4) und die Ausdehnungskammer (5) verschlossen sind, und die mit mindestens einem durchgehenden Schlitz (7) an der Kammer versehen ist, und
- eine atmungsaktive Membran (10), die zumindest an dem mindestens einen Schlitz (7) über der Trennfolie (6) angeordnet ist,
wobei die Trennfolie (6) zumindest an einem ersten umlaufenden Streifen (8), der außerhalb der Aufnahmewanne (4) liegt, an einem zweiten umlaufenden Streifen (18), der außerhalb der Ausdehnungskammer (5) liegt, und an mindestens einem Bereich (9) zum Verbinden der Wanne und der Kammer mit der tragenden Wand (3) assoziiert ist und an dieser klebt,
**dadurch gekennzeichnet, dass** er Retraktormittel (11) umfasst, die in der Aufnahmewanne (4) und/oder der Ausdehnungskammer (5) untergebracht sind, um die Trennfolie (6) und die tragende Wand (3) an dem mindestens einen Verbindungsbereich (9) voneinander zu trennen, sodass mindestens ein Kanal (12) für den Durchtritt der mit Duft versehenen Substanz (S) definiert wird, und die mittels eines Drucks, der von außen an der Aufnahmewanne (4) und/oder der Ausdehnungskammer (5) ausgeübt wird, aktivierbar sind.

2. Diffuser (1) nach Anspruch 1, **dadurch gekennzeichnet, dass** die Retraktormittel (11) mindestens einen Auftrenner (13) und mindestens ein Anlageelement (14) umfassen, die zueinander gelenkig und dafür eingerichtet sind, von einer inaktiven Konfiguration, in der sie vollständig in der Aufnahmewanne (4) und/oder der Ausdehnungskammer (5) untergebracht sind, in eine Funktionskonfiguration, in der sie zueinander eingezogen sind, überzugehen, wobei der mindestens eine Auftrenner (13) derart eingelegt ist, dass er nach der Ausübung des Drucks von außen durch den Verbindungsbereich (9) zwischen der Trennfolie (6) und der tragenden Wand (3) geht, so dass der Durchgangskanal (12) geöffnet wird.

3. Diffuser (1) nach Anspruch 2, **dadurch gekennzeichnet, dass** der mindestens eine Auftrenner (13) ein abgeschrägtes freies Ende (15) aufweist.

4. Diffuser (1) nach Anspruch 2, **dadurch gekennzeichnet, dass** das mindestens eine Anlageelement (14) ein abgerundetes freies Ende (16) aufweist.

5. Diffuser (1) nach einem oder mehreren der vorhergehenden Ansprüche, **dadurch gekennzeichnet, dass** die Retraktormittel (11) mindestens zwei Anlageelemente (14) umfassen.

6. Diffuser (1) nach einem oder mehreren der vorhergehenden Ansprüche, **dadurch gekennzeichnet, dass** die Retraktormittel (11) mindestens zwei Auftrenner (13) umfassen, wobei mindestens zwei entsprechende Verbindungsbereiche (9) bereitgestellt sind.

7. Diffuser (1) nach einem oder mehreren der vorhergehenden Ansprüche, **dadurch gekennzeichnet, dass** die Ausdehnungskammer (5) am Umfangsbereich der Aufnahmewanne (4) angeordnet ist und dass der erste umlaufende Streifen (8) zwischen der Aufnahmewanne (4) und der Ausdehnungskammer (5) verläuft, wobei der Verbindungsbereich (9) derart definiert ist, dass er einstückig mit einem Abschnitt des ersten umlaufenden Streifens (8) ist.

8. Verfahren zum Herstellen eines Duftdiffusers (1), umfassend:
- einen Schritt des Bildens eines Hauptkörpers (2), der eine tragende Wand (3) umfasst, in der in einer eingesenkten Anordnung eine Wanne (4) zum Aufnehmen einer mit Duft versehenen Substanz (S) und eine Ausdehnungskammer (5) vorhanden sind,
- einen Schritt des Einbringens eines vordefinierten Volumens einer mit Duft versehenen Substanz (S) in die Aufnahmewanne (4),
- einen ersten Schritt des Auflegens einer Trennfolie (6) auf den Hauptkörper (2) angeordnet ist, so dass die Aufnahmewanne (4) und die Ausdehnungskammer (5) verschlossen werden, wobei die Trennfolie (6) mit mindestens einem durchgehenden Schlitz (7) versehen ist, der sich an der Ausdehnungskammer (5) befindet,
- einen ersten Schritt des Anklebens der Trennfolie (6) und der tragenden Wand (3) aneinander zumindest an einem ersten umlaufenden Streifen (8), der außerhalb der Aufnahmewanne (4) liegt, an einem zweiten umlaufenden Streifen (18), der außerhalb der Ausdehnungskammer (5) liegt, und an mindestens einem Verbindungsbereich (9) zwischen der Wanne und der Kammer,
- einen zweiten Schritt des Auflegens einer atmungsaktiven Membran (10) auf die Trennfolie (6) zumindest an dem Schlitz (7),
- einen zweiten Schritt des Anklebens der atmungsaktiven Membran (10) und der Trennfolie (6) aneinander,
**dadurch gekennzeichnet, dass** es einen Schritt des Platzierens von Retraktormitteln (11) in der Aufnahmewanne (4) und/oder der Ausdehnungskammer (5), der vor dem ersten Auflegeschritt durchgeführt wird, wobei die Retraktormittel (11) dafür eingerichtet sind, die Trennfolie (6) und die tragende Wand (3) an dem mindestens einen Verbindungsbereich (9) voneinander zu trennen, sodass mindestens ein Kanal (12) für den Durchtritt der mit Duft versehenen Substanz (S) definiert wird, und mittels eines Drucks, der von außen ausgeübt wird, aktivierbar sind.

## Revendications

1. Diffuseur (1) de parfums avec une activation facilitée, qui comprend :
- un corps principal (2) doté d'une paroi de support (3) dans lequel figure, dans un agencement en retrait, un bac (4) pour collecter une substance parfumée (S) et une chambre d'expansion (5),
- un film de séparation (6) qui est superposé sur ledit corps principal (2) afin de fermer ledit bac de collecte (4) et ladite chambre d'expansion (5), et qui est doté d'au moins une encoche traversante (7) au niveau de ladite chambre, et
- une membrane respirante (10) superposée sur ledit film de séparation (6) au moins au niveau d'au moins ladite encoche traversante (7),
le film de séparation (6) étant associé et adhérant à la paroi de support (3) au moins au niveau d'une première bande périphérique (8) qui est externe au bac de collecte (4), au niveau d'une seconde bande périphérique (18) qui est externe à la chambre d'expansion (5) et au niveau d'au moins une zone (9) pour la connexion entre ledit bac et ladite chambre,
**caractérisé en ce qu'**il comprend des moyens de rétraction (11) qui sont disposés au niveau d'un ou des deux bacs de collecte (4) et de la chambre d'expansion (5) afin de détacher ledit film de séparation (6) et ladite paroi de support (3) au niveau d'au moins une zone de connexion (9) afin de délimiter au moins un canal (12) pour le passage de la substance parfumée (S), qui est activable au moyen d'une pression appliquée depuis l'extérieur au niveau dudit ou desdits deux bacs de collecte (4) et de la chambre d'expansion (5).

2. Diffuseur (1) selon la revendication 1, **caractérisé en ce que** lesdits moyens de rétraction (11) comprennent au moins un séparateur (13) et au moins un élément de butée (14), qui sont mutuellement articulés et sont conçus pour passer d'une configuration inactive, dans laquelle ils sont totalement placés dans l'un ou les deux bacs de collecte (4) et la chambre d'expansion (5), à une configuration de fonctionnement, dans laquelle ils sont mutuellement rétractés, avec au moins le séparateur (13) inséré afin de passer à travers la zone de connexion (9) entre le film de séparation (6) et la paroi de support (3) pour ouvrir le canal de passage (12), suite à l'application de ladite pression depuis l'extérieur.

3. Diffuseur (1) selon la revendication 2, **caractérisé en ce que** au moins ledit séparateur (13) dispose d'une extrémité libre effilée (15).

4. Diffuseur (1) selon la revendication 2, **caractérisé en ce que** au moins ledit élément de butée (14) dispose d'une extrémité libre arrondie (16).

5. Diffuseur (1) selon l'une ou plusieurs des revendications précédentes, **caractérisé en ce que** lesdits moyens de rétraction (11) comprennent au moins deux éléments de butée (14).

6. Diffuseur (1) selon l'une ou plusieurs des revendications précédentes, **caractérisé en ce que** lesdits moyens de rétraction (11) comprennent au moins deux séparateurs (13), au moins deux zones de connexion (9) respectives étant fournies.

7. Diffuseur (1) selon l'une ou plusieurs des revendications précédentes, **caractérisé en ce que** ladite chambre d'expansion (5) est disposée sur la zone périphérique dudit bac de collecte (4) et **en ce que** ladite première bande périphérique (8) s'étend entre ledit bac de collecte (4) et ladite chambre d'expansion (5), la zone de connexion (9) étant délimitée afin d'être intégrée à une partie de la première bande périphérique (8).

8. Méthode pour la fabrication d'un diffuseur de parfum (1), comprenant :
- une étape pour le façonnage d'un corps principal (2) comprenant une paroi de support (3) dans lequel figurent, dans un agencement en retrait, un bac (4) pour collecter une substance parfumée (S) et une chambre d'expansion (5),
- une étape pour l'introduction dans ledit bac de collecte (4) d'un volume prédéfini de substance parfumée (S),
- une première étape pour la juxtaposition d'un film de séparation (6) sur ledit corps principal (2) afin de fermer ledit bac de collecte (4) et ladite chambre d'expansion (5), le film de séparation (6) étant doté d'au moins une encoche traversante (7) située au niveau de la chambre d'expansion (5),
- une première étape pour l'adhésion dudit film de séparation (6) et de ladite paroi de support (3) au moins au niveau d'une première bande périphérique (8) qui est externe audit bac de collecte (4), au niveau d'une seconde bande périphérique (18) qui est externe à ladite chambre d'expansion (5), et au niveau d'au moins une zone de connexion (9) entre ledit bac et ladite chambre,
- une seconde étape pour la juxtaposition d'une membrane respirante (10) sur ledit film de séparation (6) au moins au niveau de ladite encoche (7),
- une seconde étape pour l'adhésion de ladite membrane respirante (10) et dudit film de séparation (6),
**caractérisée en ce qu'**elle comprend une étape pour le placement des moyens de rétraction (11) au sein d'un ou des deux bacs de collecte (4) et de la chambre d'expansion (5), qui est effectuée avant ladite première étape de juxtaposition, les moyens de rétraction (11) étant conçus pour détacher le film de séparation (6) et la paroi de support (3) au niveau d'au moins ladite une zone de connexion (9) afin de délimiter au moins un canal (12) pour le passage de la substance parfumée (S) et étant activables au moyen d'un pression appliquée depuis l'extérieur.
